# FASCICULE DE BREVET EUROPEEN

(11) **EP 3 277 234 B1**
(45) Date de publication et mention de la délivrance du brevet: **13.10.2021**
(21) Numéro de dépôt: 16721922.9
(22) Date de dépôt: 31.03.2016
(51) Int. Cl.: A61F 9/00, B65D 47/18

(54) **DISPOSITIF DE DISTRIBUTION DE LIQUIDE HORS D'UN FLACON DE CONDITIONNEMENT STÉRILE**
VORRICHTUNG ZUR ABGABE VON FLÜSSIGKEIT AUS EINER STERILEN VERPACKUNGSFLASCHE
DEVICE FOR DISPENSING LIQUID FROM A STERILE PACKAGING BOTTLE

(30) Priorité: 31.03.2015 WO PCT/IB2015/000423
(43) Date de publication de la demande: 07.02.2018
(73) Titulaire: Laboratoires THEA, 63100 Clermont-Ferrand (FR)
(72) Inventeur: DEFEMME, Alain, 63400 Chamalières (FR); MERCIER, Fabrice, 63000 Clermont-Ferrand (FR)
(74) Mandataire: Santarelli
(86) Numéro de dépôt international: PCT/IB2016/000408
(87) Numéro de publication internationale: WO 2016/156968

(56) Documents cités:
- WO-A1-2006/000897
- WO-A1-2011/095877
- US-A- 5 681 468

## Description

L'invention concerne les dispositifs de distribution de liquide qui sont utilisés dans les techniques du flaconnage pour le conditionnement de produits qui doivent être conservés à l'état stérile, non seulement jusqu'à l'ouverture du flacon, mais ensuite tant que dure la consommation du produit jusqu'à l'épuisement complet du contenu du flacon.

En exemple typique des besoins que l'invention vise à satisfaire, on se placera dans le domaine des flacons multidoses, recevant des solutions aqueuses à distribuer de manière discontinue, par doses étagées dans le temps, qui sont équipés de membranes d'interface air/liquide faisant obstacle au passage des contaminants microbiologiques de l'air ambiant dans le flacon par effet de filtration.

On connaît aussi de telles membranes qui ont en plus la particularité d'être doublement sélectivement perméables, en se laissant traverser préférentiellement soit par l'air soit par le liquide en fonction d'un différentiel de pression s'exerçant entre ses deux faces, alternativement dans le sens amont vers aval lors de la phase d'expulsion d'une dose de liquide hors du flacon et dans le sens aval vers amont lors de la phase d'aspiration quand de l'air est appelé à rentrer dans le flacon en compensation volumique de ce qui en a été extrait. On trouvera décrit dans les brevets existants de la Demanderesse comment de telles membranes, dites bifonctionnelles (bifonctionnelles du point de vue des transports de flux liquides ou gazeux), sont utilisées pour assurer une circulation alternée entre liquide et air au travers d'un canal capillaire d'expulsion du liquide disposé en suite de la membrane. De telles membranes faisant office d'interface entre l'espace fermé d'un flacon de conditionnement stérile d'un liquide aqueux (une solution aqueuse d'un principe pharmacologiquement actif en particulier) sont réalisées pour partie en un matériau de nature hydrophile, sur une première zone de l'étendue totale d'interface, et pour partie en un matériau de nature hydrophobe, sur une seconde zone de la même étendue. Le fonctionnement d'une membrane ainsi réalisée est décrit notamment dans le brevet français publié à l'état de demande sous le numéro FR2872137 (demande internationale correspondante WO 2006 000897), pour une membrane disposée en travers d'un conduit unique livrant passage aux flux d'air et de liquide dans un sens et dans l'autre entre intérieur et extérieur d'un flacon à paroi élastiquement déformable manipulé pour faire alterner expulsion et aspiration.

Dans un tel contexte, l'invention vise à fournir un dispositif de distribution de liquide avec protection microbiologique présentant une sécurité élevée vis-à-vis à la fois de la stérilité microbienne et de la toxicité chimique dans son application à des flacons de conditionnement de produits liquides stériles chez lesquels la stérilité doit être préservée tout au long de la consommation du contenu du flacon, au fil d'opérations de distribution successives s'étageant dans le temps. Permettre des durées importantes de consommation progressive est un objectif majeur que l'on cherche à atteindre par là, un autre étant de permettre un conditionnement multidose pour des produits pharmaceutiques ou parapharmaceutiques s'appliquant sur des terrains hautement contaminés.

Avec ces objectifs en vue, l'invention propose d'avoir recours à une membrane bifonctionnelle hydrophile-hydrophobe qui est en outre chargée dans sa masse d'un agent biocide par effet d'oxydation ionique. Un tel agent est apporté plus particulièrement par des macromolécules porteuses d'ions métalliques à charge positive, telles que celles qu'un art antérieur désormais bien connu propose sous la forme de polymères minéraux de la famille des alumino-silicates, appelés zéolithes, retenant en leur sein des cations métalliques labiles. Parmi les ions utiles, ce sont les ions argent (Ag+ ou Ag++) qui se sont révélés le plus avantageux dans le cadre industriel des membranes de protection anti-bactéries mises en œuvre suivant la présente invention.

Dans un distributeur de liquide suivant l'invention, une telle membrane est utilisée comme source permanente d'ions métalliques biocides en combinaison avec une masse poreuse interposée sur le trajet des fluides en amont de la membrane, masse qui est constituée apte à retenir en son sein les ions biocides qui lui parviennent pour avoir été extraits de la membrane lors de la phase aspiration à chaque opération de distribution d'une dose de liquide, en constituant ainsi une réserve secondaire d'ions actifs tout en faisant, à l'égard du transport de ces ions, un tampon évitant qu'ils puissent atteindre l'espace récepteur de liquide intérieur au flacon. En pratique, on a pu observer que les ions ainsi mis en réserve, quand ils ne sont pas consommés sur place, sont aisément libérés et entraînés en retour vers la membrane lors de la phase expulsion d'une opération de distribution subséquente.

Des membranes d'interface eau/air chargées de cations métalliques à effet biocide sont connues depuis fort longtemps, comme en témoigne par exemple le brevet américain US 5 681 468, déposé en 1993 et publié en 1997. Mais jamais il n'avait été envisagé que les cations biocides puissent agir autrement qu'en attaquant les bactéries contaminant le liquide expulsé quand il se trouve en aval de la membrane après avoir traversé celle-ci. Jamais non plus il n'avait été proposé de monter la membrane comme le prévoit l'invention, dans un dispositif associant la membrane à une masse poreuse de rétention des mêmes ions actifs que ceux dont la membrane est chargée, ainsi qu'à des moyens d'organisation de la circulation des fluides à travers eux assurant l'alternance des flux au niveau de la membrane et dans la zone aval du dispositif.

Dans la mise en œuvre pratique de l'invention, cette masse poreuse est conçue sous la forme d'un insert monté dans le dispositif de distribution de liquide, en amont de la membrane, comme un bouchon de fermeture non étanche du conduit de communication entre intérieur et extérieur du flacon. Par sa porosité et sa disposition, l'insert est alors avantageusement conçu de manière à jouer le rôle des tampons régulateurs de flux que l'on connaît des flacons de gouttes ophtalmiques décrits dans les brevets antérieurs de la Demanderesse, grâce au fait qu'ils imposent une perte de charge sur le trajet du liquide poussé hors du flacon.

En revanche, pour qu'un tel insert joue son rôle dans la protection contre les polluants de la stérilité tel que prévu suivant la présente invention, il est spécialement réalisé en une matière polymère présentant des sites actifs à charge négative, aptes de ce fait à attraire les cations métalliques biocides dont la membrane est initialement chargée. Les matériaux préférés de ce point de vue sont constitués de polymères à base de polyoléfines copolymérisées avec des composés à fonction acide carboxylique. Suivant les proportions relatives des constituants et suivant les conditions dans lesquelles se déroulent les réactions de copolymérisation, il subsiste dans le polymère obtenu une proportion non négligeable de sites carboxyle libres prêts à se lier avec les cations utilisés comme cations biocides qui viennent en contact avec le polymère.

Suivant un mode de mise en œuvre préféré de l'invention la capacité spécifique de la matière polymère en rétention des cations métalliques peut être accrue en soumettant le polymère à un traitement d'irradiation ayant pour effet de libérer d'autres groupes carboxyle.

Le mode de fonctionnement du dispositif suivant l'invention dans son ensemble sera précisé dans la suite de la présente description en se référant au cas où il équipe un flacon de conditionnement stérile d'une solution ophtalmique à paroi souple, élastiquement déformable en compression du volume du réservoir interne. On doit comprendre cependant que d'autres moyens peuvent permettre d'assurer de manière similaire les variations de pression provoquant à chaque opération de distribution d'une dose de liquide d'abord une phase de propulsion de l'intérieur vers l'extérieur du flacon et d'expulsion du liquide au-delà du canal capillaire situé en aval de la membrane, puis une phase d'aspiration appelant de l'air extérieur à rentrer dans le flacon, l'air étant alors précédé d'un reflux de liquide non expulsé. On peut penser notamment à un flacon à fond mobile axialement à l'encontre d'un moyen de rappel élastique ou à un flacon équipé d'un système de pompe. D'autre part, on se référera préférentiellement à un dispositif distributeur de gouttes, mais il doit être entendu que le dispositif suivant l'invention peut s'adapter à la distribution de doses individuelles plus importantes que des gouttes, ainsi qu'à un débouché du canal capillaire diffusant le liquide sous d'autres formes, par exemple sous forme d'un jet ou avec diffusion spatiale.

Initialement, pendant toute la durée de stockage précédant la première utilisation, le flacon reste hermétiquement fermé sur une couverture d'air stérile pressurisé surmontant l'espace récepteur du liquide, si bien que la membrane reste sèche. Elle ne deviendra imbibée de liquide dans sa zone hydrophile qu'à l'occasion de la première expulsion de liquide après ouverture du flacon.

L'espace aval dudit dispositif comprend un canal capillaire où flux liquide et flux gazeux circulent en alternance, sans jamais se mélanger, de sorte qu'en fonctionnement quand ledit canal a terminé d'acheminer le flux de liquide à expulser vers l'extérieur, il reste un reliquat de liquide non expulsé qui occupe temporairement ledit canal. Ce reliquat est reconduit en reflux à travers la membrane sous la poussée du flux d'air aspiré depuis l'extérieur lorsque la différence de pression entre les deux faces de la membrane cesse de s'exercer dans le sens de l'expulsion. Dans cette phase d'aspiration, le liquide reflué passe par la zone hydrophile de la membrane, tandis que l'air rentrant en compensation du volume de liquide distribué passe par la zone hydrophobe.

En amont de la membrane, l'espace ménagé dans le dispositif suivant l'invention forme un conduit qui contrairement au canal capillaire aval, est de large section transversale. C'est dans ce conduit qu'est disposé l'insert poreux fournisseur de charges négatives dans les réactions qui tendent à retenir les cations métalliques biocides véhiculés par le liquide dans des liaisons chimiques de charges avec le polymère de l'insert au niveau des sites actifs qu'il présente notamment sous la forme de groupes carboxyles libres. Cet insert, également appelé tampon en français ou "plug" en anglais, se situe là en présence à la fois des flux de liquide et d'air, qui viennent ensemble au contact du polymère le constituant dans les cellules de la matière poreuse. Le contact se produit sur une surface importante, en correspondance avec la surface spécifique de la matière poreuse. Lorsque le dispositif est en fonctionnement, l'insert poreux retient suffisamment les cations métalliques biocides pour que le liquide en réserve dans le flacon ne puisse pas être contaminé chimiquement par des cations biocides. D'autre part il assure que s'établissent des mouvements de va-et-vient de cations biocides véhiculés par les flux et reflux de liquide, notamment en aller et retour entre la membrane et l'insert poreux, suivant un phénomène qui est propice à une haute activité biocide dans ledit dispositif de distribution du liquide tout en préservant le liquide en réserve d'une contamination microbiologique.

En effet, de manière surprenante, les inventeurs ont mis en évidence que ledit dispositif selon l'invention, conserve une forte activité biocide pendant tout le temps de son utilisation en distribution discontinue de liquide. Comme cela sera détaillé plus loin, il a été montré que le dispositif de distribution selon l'invention utilisé monté étanche en fermeture d'un flacon pour réaliser un flacon multidose, contenant par exemple un collyre stérile, présente une grande efficacité en termes de stérilité au cours de la consommation du collyre. La consommation du contenu peut ainsi s'étaler dans le temps beaucoup plus longtemps qu'avec les flacons actuels et en toute sécurité concernant l'absence de nocivité pour le patient.

Le flux d'air entrant en compensation du liquide expulsé, qui provient de l'air ambiant chargé de microorganismes, est stérilisé principalement lors de son passage à travers la membrane par action biocide de contact des cations biocides dans les pores de la membrane en sa partie hydrophobe, et le cas échéant par filtration antibactérienne. Au surplus, si besoin, du fait que des cations biocides véhiculés par le reflux du reliquat de liquide non expulsé sont retenus dans l'insert en fin de chaque distribution de liquide, il y a toujours de l'agent biocide actif disponible pour détruire les microorganismes de l'air qui stagne dans ledit insert en mélange avec une partie du reliquat de liquide.

Des essais décrits plus loin confirment que des cations biocides sont recueillis progressivement dans l'insert poreux, suivant un gradient en quantité décroissante allant de la partie extrême la plus proche de la membrane, dite ici proximale, vers la partie extrême opposée la plus proche de la réserve de liquide, dite ici partie distale, de sorte que la réserve de liquide reste exempte de cation biocides.

En outre, après la première mise en fonctionnement dudit dispositif par distribution de liquide, ledit insert poreux qui se charge en cations biocides forme alors une source en cations biocides qui peuvent être en partie extraits au passage du flux de liquide sortant de l'intérieur du flacon à travers ledit insert pour rejoindre des sites disponibles sur la membrane.

Il se crée ainsi un va-et-vient de cations biocides dans le conduit de circulation des fluides lié au va-et-vient du liquide qui maintient une quantité relativement stable, au cours des utilisations, en cations biocides disponibles au sein du dispositif selon l'invention pour être actifs sur les microorganismes arrivant à leur contact.

Dans son principe, l'invention apparaît ainsi comme consistant à réaliser la membrane d'interface eau/air bifonctionnelle d'une part, l'insert tampon installé en bouchon non étanche du flacon d'autre part, de telle manière qu'en fonctionnement, après l'ouverture du flacon pour une première utilisation en distribution de liquide, la membrane et l'insert coopèrent pour créer entre eux un lit d'ions mobiles qui sont prélevés de l'insert par le flux de liquide extrait du flacon à chaque opération de distribution (lors de la phase d'expulsion de liquide) à partir de ceux qui y ont été amenés par un reflux de liquide non distribué lors d'opérations de distribution de liquide précédentes (lors de la phase d'aspiration d'air).

Globalement, on peut admettre que la quantité d'ions métalliques biocides qui est effectivement consommée en destruction de contaminants biologiques est très faible en regard de celle qui est déplacée à chaque opération de distribution de liquide, laquelle est elle-même très faible en regard de la capacité initiale de la membrane. La quantité consommée est fonction du degré de contamination de l'air ambiant aspiré pour une efficacité de traitement de l'air qui sera d'autant meilleure que la surface de contact avec les matériaux chargés sera plus importante. La quantité déplacée est tributaire du débit de liquide assurant l'entraînement de la charge cationique active, ou plus exactement de la masse de liquide déplacée à chaque reflux de liquide de la membrane à l'insert et à chaque flux direct en expulsion vers la membrane.

Avec ces considérations à l'esprit, on pourra adapter le dispositif de distribution de liquide suivant l'invention à des applications dans des milieux plus ou moins contaminants, même dans des conditions sévères en termes de volume global de solution à distribuer, de durée totale d'utilisation du flacon, de fréquence de répétition dès opérations de distribution, en jouant sur les formes et dimensions respectives de la membrane et de l'insert poreux, à supposer que les matériaux constituant chacun d'eux restent inchangés.

En ce qui concerne la membrane elle-même, la présente invention prévoit avantageusement qu'elle soit réalisée à partir d'un matériau poreux en matière polymère de nature hydrophile chargée de manière homogène en agent à effet biocide par oxydation ionique, ledit matériau constituant ladite membrane dans toute sa masse et étant ensuite, localement sur une partie de l'étendue de la membrane venant en travers du conduit de circulation des fluides entre intérieur et extérieur du flacon, rendu hydrophobe par un traitement de polymérisation complémentaire préservant son activité biocide.

Ceci permet de ménager un volume approprié pour la mise en contact de la phase gazeuse, constituée par de l'air, avec la matière polymère chargée d'ions actifs par effet biocide au sein de la masse poreuse sur toute l'épaisseur de la membrane. Dans le même sens joue le fait que le matériau de la base hydrophile de la membrane est constitué finement homogène, ce qui exclut les réalisations antérieures des membranes filtres faites d'une matière à base fibreuse retenant entre les fibres des particules chargées. On préfère suivant l'invention partir d'une base polymère fondue comprenant des granules fusibles d'un mélange maître intégrant lui-même des macromolécules minérales porteuses des ions actifs par effet biocide.

Alors que classiquement, la filtration des bactéries demande une porosité fine, ne dépassant pas 0,2 µm, la présence d'un agent biocide au sein de la membrane permet de préserver la stérilité de manière satisfaisante avec des porosités plus grossières, préférentiellement d'environ 0,3 ou 0,4 µm, ou allant plus largement jusqu'à 0,5 µm, ou même jusqu'à 0,6 ou 0,8 µm, voire 1 µm, ce qui est avantageux du point de vue des pertes de charge et permet le traitement de liquides visqueux. En pratique, l'invention prévoit ainsi, suivant un mode de mise en œuvre préféré, de réaliser la membrane telle qu'elle présente un diamètre de pores moyen adapté à la filtration des micro-organismes de dimensions supérieures à une taille de particules comprise entre 0,3 et 1 µm, en particulier entre 0,3 et 0,6 micromètre. Au total, la porosité de la membrane pourra ainsi être ajustée à toute valeur entre 0,1 et 1 micromètre en fonction des propriétés physicochimiques du liquide.

Les macromolécules support des ions biocides sont avantageusement, ainsi qu'on l'a déjà indiqué, des polymères minéraux du type des alumino-silicates dans lesquelles les ions biocides sont intégrés, s'agissant plus spécialement, de manière en elle-même connue, d'ions métalliques comme les ions argent ou les métaux similaires sous forme ionique, qui se fixent sur les sites libres des chaînes polysiloxaniques par des liaisons covalentes polaires. Ces polymères minéraux sont de préférence des polymères cristallins. La concentration en ions actifs dans la membrane est de manière préférée, bien que non limitative des conditions d'application de l'invention, choisie comprise entre 100 et 100 000 ppm, en prenant comme exemple le cas d'un polymère minéral à base d'alumino-silicates porteur d'ions argent dans une membrane de porosité d'environ 0,2-0,3 micromètre d'étendue efficace de l'ordre de 3 cm2.

Parmi les ions métalliques utiles à l'invention, on peut retenir les ions du cuivre, du zinc mais ce sont les ions de l'argent qui se sont révélés les plus avantageux dans le cadre industriel du dispositif de protection antimicrobien mis en œuvre suivant la présente invention.

En caractéristique secondaire l'invention s'étend, au-delà du dispositif de distribution de liquide suivant l'invention, à un flacon de conditionnement stérile en faisant application, à utiliser notamment dans le cadre du conditionnement stérile de produits pharmaceutiques ou parapharmaceutiques.

L'invention concerne aussi un procédé particulier de fabrication de la membrane elle-même.

Avantageusement des moyens d'organisation de la circulation des fluides viennent compléter le dispositif de distribution monté sur un flacon de conditionnement. Préférentiellement ledit flacon de conditionnement présente une paroi à déformation élastiquement réversible pour assurer la rentrée d'air extérieur en compensation de toute dose de liquide expulsée du flacon ainsi que le reflux en retour à travers ledit dispositif de tout reliquat de liquide non expulsé, ladite membrane étant montée avec ledit insert poreux dans ledit dispositif de distribution du liquide en association avec des moyens d'organisation de la circulation des fluides air et liquide à travers elle, et dans lequel ladite membrane est disposée à la base d'un embout compte-gouttes comprenant le canal capillaire d'expulsion des gouttes, face à une embase dudit embout dans laquelle sont ménagés des moyens de guidage respectifs de l'air aspiré depuis l'extérieur et de tout reliquat de liquide non distribué appelé à refluer à travers la membrane de l'espace aval vers l'espace amont, lesdits moyens tendant à diriger le flux d'air vers la partie hydrophobe de la membrane disposée de préférence au centre de ladite membrane et à répartir le liquide sur sa partie hydrophile.

### Description d'un mode de réalisation de flacon

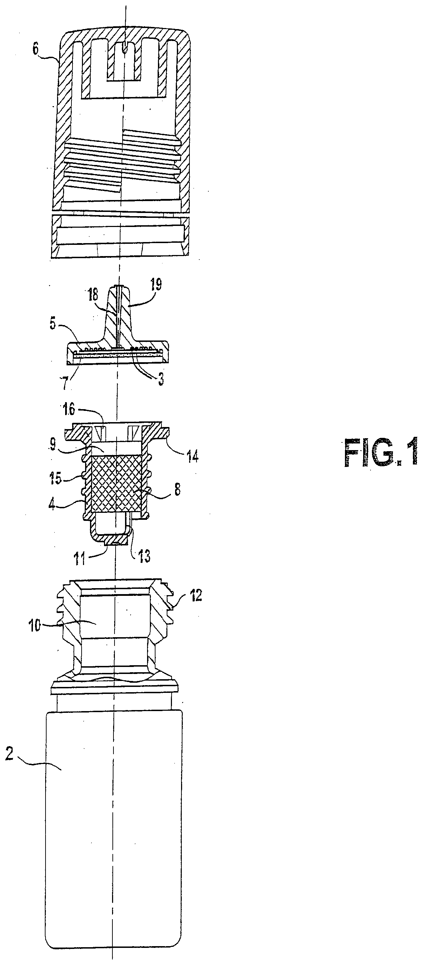

L'invention sera maintenant plus complètement décrite dans le cadre de caractéristiques préférées et de leurs avantages, suivant des exemples non limitatifs, en se référant à un dispositif de distribution d'un liquide stérile suivant l'invention dans son application à la préservation de la stérilité dans un flacon à embout compte-gouttes tel qu'illustré sur les figures 1 à 4, dans lesquelles :
- la figure 1 représente, en coupe longitudinale et en vue éclatée, les différents éléments d'un flacon à paroi déformable d'où un liquide est expulsé par doses successives à travers un dispositif de distribution de protection microbiologique selon l'invention;
- la coupe longitudinale de la figure 2 représente plus particulièrement le dispositif de distribution, une fois assemblés ses éléments propres pour constituer une tête de distribution de liquide et rentrée d'air extérieur s'insérant dans le col du flacon ;
- la figure 3 illustre la configuration de l'embase de l'embout en sa surface venant en regard de la nacelle du dispositif illustré en figures 1 ou 2 ;
- la figure 4 illustre, en vue partiellement éclatée, la circulation des fluides en retour dans le conduit de circulation du dispositif de distribution selon l'invention représenté en figures 1 ou 2.

Dans sa constitution générale et tel qu'il est représenté dans tous ses éléments sur la figure 1, le flacon équipé d'une tête de distribution apparaît conforme à la conception usuelle des flacons de conditionnement stérile. Le flacon comprend un réservoir 2 recevant le liquide aqueux à distribuer à l'état stérile surmonté d'un dispositif de distribution monté étanche dans le col du flacon 10. Mais il en diffère par des caractéristiques propres à l'invention qui se répartissent sur ses éléments constitutifs essentiels pour la distribution sous protection microbiologique assurée suivant l'invention, le long du conduit de circulation du liquide aqueux à distribuer et de l'air entrant en compensation du liquide expulsé, à savoir principalement en partie amont de ce conduit l'insert poreux 8 sous forme d'un tampon occupant l'espace interne dans la nacelle 4, et à l'interface de ces parties aval et amont la membrane 7. Il en diffère aussi dans leur assemblage relatif lié à la circulation des fluides et aux effets sur l'activité biocide dans le conduit qui en résultent.

Conformément à l'invention, la membrane à perméabilité sélective que comporte le dispositif de distribution est utilisée en séparation des flux de liquide et d'air qui la traversent, en membrane de protection microbiologique par filtration et, par sa matière contenant des macromolécules minérales porteuses de cations biocides, en destruction des bactéries ou micro-organismes analogues véhiculés dans les fluides qui la traversent.

Dans l'exemple choisi pour illustrer au mieux l'invention, la membrane est de base en polymère organique, plus précisément dans le cas présent à base de résine de polyester modifiée par une résine de polyamide ou de polyéther-sulfone, à laquelle ont été intégrées en masse les macromolécules minérales support des cations biocides, plus particulièrement ici une zéolithe à charge cationique d'argent. Elle est de nature hydrophile et elle est rendue hydrophobe sur une partie seulement de son étendue en travers du conduit ménagé dans le dispositif de distribution. Par exemple, on procède par exposition locale à irradiation sous rayonnement ultraviolet, ce qui modifie la structure du polymère in situ, par des réactions de réticulation radicalaire entre ses constituants, en préservant les propriétés des cations biocides des zéolithes.

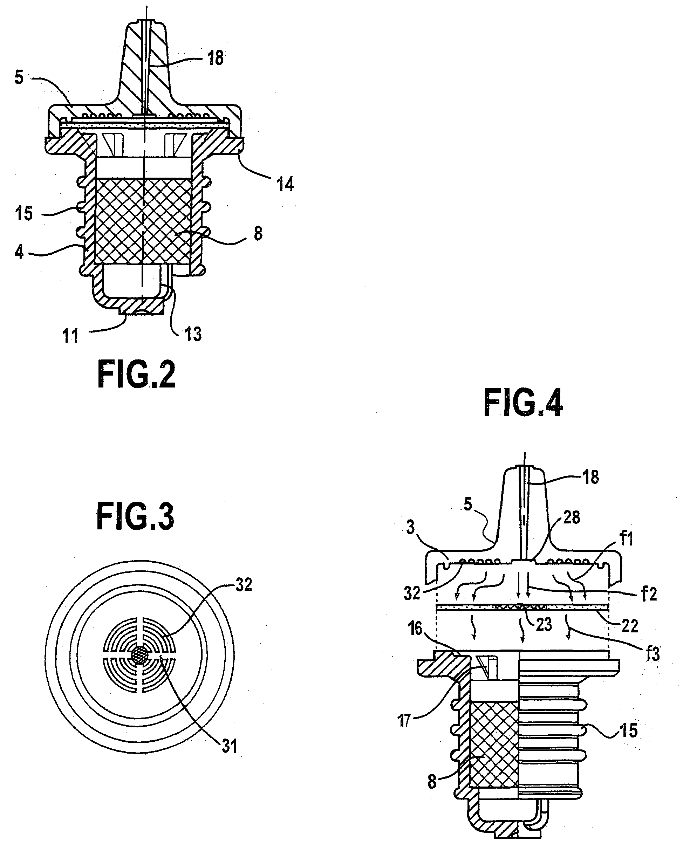

La membrane illustrée sur la figure 4 présente ainsi une zone hydrophile 22 se laissant traverser préférentiellement par le liquide aqueux en présence d'air et une zone hydrophobe 23 se laissant traverser préférentiellement par l'air en présence d'eau ou du liquide aqueux. La charge ionique à effet biocide est présente aussi bien dans la zone hydrophobe que dans la zone hydrophile.

En fonctionnement, lors des opérations successives de distribution des doses de liquide hors du flacon, étagées dans le temps, la structure de la membrane, en liaison avec l'organisation de la circulation des fluides à travers elle, tend à favoriser une action destructrice des micro-organismes qui s'exerce sur le flux d'air au sein même de la matière hydrophobe, par contact entre l'air et le polymère chargé d'ions à la surface des pores, alors qu'au contraire, dans la partie hydrophile de la membrane, les ions biocides ne sont pas consommés mais entraînés et véhiculés plus loin par le liquide traversant la membrane.

A l'intérieur du conduit de circulation des fluides, en amont de la membrane 7 du côté de l'espace intérieur fermé du flacon, on trouve un insert poreux 8 qui a pour rôle principal, conformément à l'invention, de retenir les cations biocides que lui amène chaque reflux de liquide aqueux constitué du reliquat non expulsé de la dose de liquide précédemment soutirée du flacon, et de laisser repartir vers la membrane des cations biocides dont il s'est chargé quand ensuite on expulse une nouvelle dose de liquide hors du flacon.

Dans des exemples classiques dans le cadre des applications ophtalmiques, l'insert poreux a une longueur suivant l'axe du flacon de 9 mm et un diamètre de 9,6 mm. Plus généralement et à titre indicatif, ledit insert peut avoir une longueur comprise entre 5 à 15 mm. Les dimensions de l'insert sont adaptées à la taille de son récepteur.

Sa distance à la membrane considérée sur sa face amont, est du même ordre de grandeur. Sa porosité correspond à un débit d'air de 3 000 ml/mn, tel que mesuré suivant la méthode capacitive du "water flow", consistant à mesurer le temps de remplissage d'un volume donné à l'aide d'un chronomètre. Sa masse volumique est selon l'exemple de l'ordre de 0,50 g.cm3.

Plus généralement l'insert qui comprend un grand nombre de cellules ouvertes, présente une porosité correspondant de préférence à un débit d'air compris entre 1 000 et 4 000 ml/min, mesuré selon la méthode capacitive du « water flow ». Sa masse volumique est de préférence comprise entre 0,20 et 0,80 g.cm3.

### Tests d'acidité de l'insert poreux

L'insert poreux destiné à un flacon pour liquide ophtalmique selon l'exemple considéré ici est réalisé à partir d'un filament extrudé en polymère à base de polyéthylène qui est soumis à compactage. Le polymère comporte des groupes carboxyliques initialement dans la mesure où l'éthylène a été copolymérisé avec des composés à fonctions acide carboxylique, constitués par exemple ici d'homologues supérieurs (à chaîne hydrocarbonée en C4-C10) d'acide carboxyliques, dans une proportion d'au plus 25 %.

A ce stade il comporte déjà des sites carboxyles laissés libres par les réactions de polymérisation. C'est ce qui explique les résultats de tests rapportés plus loin qui permettent de mettre l'effet sur les cations en relation avec l'acidité mesurée dans l'insert.

La proportion de sites carboxyles libres peut être augmentée en exposant le produit à un rayonnement capable de casser les molécules du polymère. Des rayons bêta ou gamma sont appropriés pour cela.

A titre d'exemple, l'insert compacté est soumis, en présence d'air, à une irradiation par des rayons gamma (source Cobalt 60, 25 kGy). Les radicaux formés dans la matière polymère lors de l'irradiation réagissent avec l'air pour former notamment des groupes anioniques carboxyle.

La teneur en sites carboxyles avant et après irradiation est étudiée par des mesures d'acidité qui sont réalisées selon le principe de dosage de l'acidité ou l'alcalinité de la Pharmacopée Européenne 8.6 pour les polyoléfines. Ces mesures sont effectuées par comparaison avec l'eau purifiée, sur de l'eau chauffée avec des inserts non irradiés et sur de l'eau chauffée avec des inserts irradiés.

Les résultats sont présentés dans le tableau 1 ci-après.

**Tableau 1 :**

| | Eau purifiée | Inserts non irradiés | Inserts irradiés |
|---|---|---|---|
| pH | 6,8 | 5,5 | 5,0 |
| Vh (ml) | 1,2 | 1,5 | 2,4 |

La baisse du pH et l'augmentation du volume équivalent (Vh) au point de virage de l'indicateur coloré témoignent de la création d'un grand nombre de sites acides dans les inserts irradiés, d'où une augmentation sensible par rapport au cas des inserts de copolymère avec des monomères à groupes fonctionnels carboxyliques non encore irradiés.

Le reliquat de liquide qui atteint le réservoir récepteur de liquide intérieur au flacon y arrive stérile et exempt de cations biocides. La preuve en est établie par les essais ci-après.

### Essais d'innocuité pour le liquide conservé stérile

Des tests ont été effectués pour déterminer la quantité d'ions d'argent retrouvés dans l'espace fermé, en amont de la membrane, d'un premier flacon contenant une solution A et d'un second flacon contenant une solution B, solutions décrites ci-dessous, à savoir dans l'insert découpé dans sa longueur en trois tranches d'épaisseurs égales formant la partie proximale de l'insert, la partie centrale de l'insert et la partie distale de l'insert, ainsi que dans la solution en réserve, à différents temps d'utilisation des flacons qui correspondent à un volume de solution extraite du flacon par expulsions intermittentes de gouttes.

Dans ces tests, deux solutions en milieu aqueux connues comme collyres sont testées : une solution physiologique A contenant comme principe actif du chlorure de sodium en milieu aqueux employée usuellement comme collyre dans le traitement de la sécheresse oculaire, et une solution ophtalmique B contenant comme principe actif du maléate de timolol en milieu aqueux employée usuellement comme collyre dans le traitement du glaucome.

Les résultats sont présentés en tableau 2 pour la solution A et en tableau 3 pour la solution B ci-après.

**Tableau 2 : Quantité d'ions argent en partie amont du conduit de circulation des fluides pour la solution A**

| Temps d'utilisation du flacon | Première utilisation | 15 jours | 30 jours | 90 jours |
|---|---|---|---|---|
| Volume de solution A extrait | 4 gouttes (0,15 ml) | 1,67 ml | 3,35 ml | 10 ml (environ 300 gouttes) |
| En partie proximale de l'insert (ppm) | 4,18 | 0,88 | 1,03 | 0,94 |
| En partie centrale de l'insert (ppm) | 1,75 | 0,56 | 0,44 | 0,61 |
| En partie distale de l'insert (ppm) | 0,81 | 0,26 | 0,31 | 0,22 |
| Dans le réservoir (ppm) | < 0,001 ppm | < 0,001 ppm | < 0,001ppm | < 0,001 ppm |

**Tableau 3 : Quantité d'ions argent en partie amont du conduit de circulation des fluides pour la solution B**

| Temps d'utilisation du flacon | Première utilisation | 15 jours | 30 jours | 90 jours |
|---|---|---|---|---|
| Volume de solution B extrait | 4 gouttes (0,15 ml) | 1,08 ml | 2,17 ml | 6,5 ml (environ 200 gouttes) |
| En partie proximale de l'insert (ppm) | 7,73 | 2,08 | 1,81 | 1,34 |
| En partie centrale de l'insert (ppm) | 4,95 | 1,36 | 0,91 | 0,63 |
| En partie distale de l'insert (ppm) | 1,08 | 0,36 | 0,42 | 0,27 |
| Dans le réservoir (ppm) | < 0,001 ppm | < 0,001ppm | < 0,001 ppm | < 0,001ppm |

Les résultats de ces tableaux 2 et 3 montrent d'une part que des ions argent sont bien retenus dans l'insert et d'autre part que la quantité d'ions d'argent retenus dans l'insert diminue de la partie proximale vers la partie distale de l'insert, alors que dans la réserve de liquide la quantité d'ions argent est inférieure au seuil de détection (0,001ppm).

La réserve de liquide est donc bien protégée d'une contamination chimique par les cations biocides.

La quantité en cations argent retenus dans l'insert est importante à la première utilisation, mais elle tend à diminuer au cours de l'utilisation prolongée du flacon, sans toutefois diminuer brutalement, ce qui montre qu'un mouvement d'échange d'ions biocides a lieu en amont de la membrane entre celle-ci en tant que source primaire de cations et l'insert en tant que zone de rétention de cations biocides acheminés par le reflux de liquide. L'insert devient alors une source secondaire de cations biocides disponibles pour être utilisés au cours des prélèvements de liquide vers la membrane.

### Essais de contamination forcée

Des essais relatifs à l'efficacité antimicrobienne du dispositif par des tests d'efficacité antimicrobienne dite forcée et au cours du temps sont réalisés d'une part avec un dispositif D1 à insert irradié tel que décrit en référence aux figures, avec d'autre part un dispositif D2 constitué comme le dispositif D1 sauf que l'insert est non irradié, en comparaison avec un dispositif D3 dont l'insert est en polyéthylène irradié comme le dispositif D1, mais dont la membrane antimicrobienne est constituée de la même matière polymère de base que celle du dispositif de l'invention mais exempte de tout agent biocide.

Le test de contamination biologique forcée consiste à simuler une utilisation du flacon par expulsion de gouttes de liquide suivie d'inoculation de germes contaminants en une quantité importante donnée, la quantité de germes retrouvés dans une goutte de solution expulsée ultérieurement étant alors déterminée. Les résultats de test présentés ci-après dans les tableaux 4 et 5 ont été déterminés en suivant le protocole suivant. Après avoir mis en service un flacon contenant une solution stérile par expulsion de quatre gouttes de cette solution, des germes contaminants en une quantité importante, ici 10⁵ (cent mille) germes, sont inoculés dans l'orifice de l'embout du flacon puis on détermine la quantité de germes présents dans une goutte de liquide expulsée 6 heures (temps T6) après cette première inoculation. Le jour suivant, soit 24 heures après la mise en service du flacon, on extrait une goutte de solution du flacon suivi d'une inoculation de 10⁵ germes dans l'embout du flacon, cette manipulation étant réalisée trois fois dans la journée, une fois le matin, une fois le midi et une fois le soir, pour simuler une utilisation habituelle d'un collyre. On extrait une goutte de solution 24 heures (temps T24) après la dernière inoculation et on détermine la quantité de germes présents dans cette goutte.

On réalise d'autre part des tests de contamination forcée sur des flacons similaires en extrayant d'abord des gouttes de solution en un volume correspondant à trois mois d'utilisation d'une solution donnée, puis on applique le protocole de contamination forcée comme ci-dessus par inoculation de 10⁵ germes dans l'orifice de l'embout et analyse d'une goutte de solution 6 heures après (au temps T6) et on poursuit le jour suivant par la manipulation d'extraction d'une goutte suivi d'une inoculation, trois fois dans la journée, et on détermine la quantité de germes présents dans une goutte extraite 24 heures après cette dernière inoculation (temps T24).

On teste les flacons avec la solution physiologique A et la solution ophtalmique B qui ont été précédemment décrites pour les exemples des tableaux 2 et 3.

Dans ces tests, deux souches de bactéries aérobies contaminantes ont été utilisées : une souche P de *Pseudomonas aeruginosa* et une souche E de *Escherichia coli.*

Les résultats sont présentés dans les tableaux 4 et 5 suivants :

**TABLEAU 4 : tests de contamination forcée avec la solution physiologique A**

| | Dispositif D1 Insert irradié | | Dispositif D2 Insert non irradié | | Dispositif D3 : Pas de cations Ag dans la membrane) | | Durée d'utilisation du flacon |
|---|---|---|---|---|---|---|---|
| Temps analyse | T6 | T24 | T6 | T24 | T6 | T24 | |
| Souche P | 8 | 2 | 1 000 | 10 | 10 000 | 100 000 | Immédiate |
| Souche P | <1 | <1 | 1 000 | 10 | 10 000 | 100 000 | A 3 mois (10 ml extraits) |
| Souche E | 100 | <1 | 10 000 | 100 | 100 000 | 100 000 | Immédiate |
| Souche E | 10 | <1 | 10 000 | 100 | 100 000 | 100 000 | A 3 mois (10 ml extraits) |

**TABLEAU 5 : tests de contamination forcée avec la solution ophtalmique B**

| | Dispositif D1 selon l'invention | | Dispositif D2 comparatif avec insert non irradié | | Dispositif D3 avec membrane non chargée initialement | | Durée d'utilisation du flacon |
|---|---|---|---|---|---|---|---|
| Temps analyse | T6 | T24 | T6 | T24 | T6 | T24 | |
| Souche P | <1 | <1 | 1 000 | 10 | 100 000 | 100 000 | Immédiate |
| Souche P | <1 | <1 | 100 | 10 | 100 000 | 100 000 | A 3 mois : (6,5 ml extraits) |
| Souche E | <1 | <1 | 1 000 | 100 | 100 000 | 100 000 | Immédiate |
| Souche E | <1 | <1 | 1 000 | 10 | 100 000 | 100 000 | A 3 mois (6,5 ml extraits) |

Les résultats de ces tableaux 4 et 5 montrent la grande efficacité du dispositif de distribution selon l'invention pour maintenir la stérilité d'un liquide, mis stérile en réserve, au cours d'une longue utilisation par doses étagées dans le temps.

Les résultats rapportés ici ont l'intérêt, en sortant des conditions normales d'utilisation des flacons de gouttes ophtalmiques, de montrer que la qualité microbiologique du liquide délivré à travers le dispositif de l'invention reste acceptable même quand on a provoqué artificiellement une contamination exceptionnellement importante. Il s'ensuit que l'on pourra utiliser le même dispositif de l'invention dans des applications impliquant des conditions beaucoup plus sévères en risque de contamination, par exemple pour des produits devant être diffusés sur des plaies, des brûlures, sur des peaux atopiques en cosmétologie, etc. Le conditionnement de tels produits en flacons multidoses devient ainsi possible grâce à l'invention. De plus, il est clair que l'on ne pouvait espérer de tels résultats avec les systèmes antérieurement connus.

### Essais avec un liquide visqueux

Les essais de contamination forcée sont ici réalisés pour convenir à une solution visqueuse, grâce à l'utilisation d'une membrane de diamètre de pores moyen nettement supérieur à 0,2 µm, choisie ici à titre d'exemple à 0,8 µm, bien supérieure à la porosité de 0,2 µm usuellement admise pour une bonne efficacité de filtration bactérienne.

On teste un dispositif selon l'invention équipée d'une membrane ayant un diamètre de pores moyens de 0,80µm en utilisation avec une solution visqueuse V en comparaison avec un dispositif selon l'invention équipée d'une membrane à diamètre de pores moyens de 0,22µm en utilisation avec une solution peu visqueuse T.

Les deux solutions sont à base d'acide hyaluronique en quantités différentes, mis en solution dans de l'eau tamponnée à un pH d'environ 7. Pour un volume total d'une solution aqueuse de 100ml, la solution visqueuse V comporte 0,30 g d'acide hyaluronique et présente une viscosité de 60 mPa.s, alors que la solution T peu visqueuse ne comporte que 0,15 g d'acide hyaluronique et présente une viscosité de 3 mPa.s .

Les tests de contamination forcée sont réalisés en suivant le même protocole que décrit précédemment et avec les deux mêmes souches de germes contaminants, pour une utilisation immédiate du flacon et pour une utilisation simulée de 3 mois. Les résultats sont présentés en tableau 6 ci-après.

Ces essais prouvent une grande efficacité du dispositif de distribution selon l'invention par effet biocide au cours du temps pour conserver la stérilité du liquide dans le flacon malgré une efficacité antibactérienne par filtration nettement moindre.

**TABLEAU 6 : tests de contamination avec les solutions T et V**

| | Dispositif selon l'invention avec membrane à 0,22 µm et solution T | | Dispositif selon l'invention avec membrane à 0,80 µm et solution visqueuse V | | Durée d'utilisation du flacon |
|---|---|---|---|---|---|
| Temps analyse | T6 | T24 | T6 | T24 | |
| Souche P | <1 | <1 | <1 | <1 | Immédiate |
| Souche P | <1 | <1 | <1 | <1 | A 3 mois (27 ml extraits) |
| Souche E | 2 | <1 | <1 | <1 | Immédiate |
| Souche E | 2 | <1 | <1 | <1 | A 3 mois (27 ml extraits) |

Les essais ci-dessus ont été réalisés en utilisant un flacon équipé d'une tête de distribution de liquide dans laquelle, conformément à l'invention, la concentration en charge ionique initiale dans la membrane, en cations d'argent, est de l'ordre de quelques milliers de ppm . Bien entendu il s'agit de cas d'exemples, que l'on pourra adapter en modifiant les données chiffrées en fonction des conditions rencontrées en pratique dans chaque cas d'application de l'invention.

### Suite de la description des figures

Selon un mode particulier de mise en œuvre de l'invention, le canal capillaire est creusé dans un embout réalisé lui-même en une matière chargée d'agent biocide, ici également apporté par une charge de zéolithe support des ions. Le canal capillaire 18 est ainsi réalisé dans un embout de matériau polymère dense, non perméable aux fluides liquide et air, chargé en cations biocides d'argent qui peuvent se déplacer en migrant depuis la masse vers la surface. Par exemple l'embout peut être en polyéthylène chargé d'agent biocide, notamment de zéolithes support de cations d'argent.

Une tête de distribution dont l'embout est ainsi chargé d'agent biocide alors que le matériau constituant la nacelle en est dépourvu, est suffisamment décrite dans la demande de brevet antérieure WO2010/013131 de la Demanderesse pour qu'il soit inutile d'en décrire plus les détails ici.

Pour compléter la description du dispositif de distribution de liquide dans son application à un flacon, en référence aux figures 3 et 4, on notera qu'en amont de la membrane dans la partie large du conduit de circulation des fluides ménagé par la forme annulaire de la nacelle 4, la surface libre de la membrane est dégagée. Toutefois, des ailettes de soutien 16, 17 sont formées sur la nacelle, du côté interne, pour limiter les contraintes pouvant s'exercer en fonctionnement sur le pourtour de la membrane, là où elle est fixée collée sur une couronne périphérique de l'embase de l'embout pourvu du canal capillaire d'expulsion de liquide, mais elles laissent la membrane libre de se bomber à l'écart de l'embase 3 de l'embout.

En regard de la face externe de la membrane vue en sa nature hydrophile, l'embase 3 de l'embout 5 forme une surface d'appui pour la membrane dans les phases d'expulsion de liquide, qui rejoint la paroi du canal capillaire 18 au niveau de son embouchure évasée 28.

Autour de cette embouchure, la surface libre de l'embout est creusée de rainures radiales offrant une large section de passage au liquide au voisinage de la membrane du côté extérieur au flacon. Ces rainures en disposition rayonnantes 31 ont pour rôle de collecter le liquide sortant du flacon en le guidant vers l'embouchure du canal capillaire 18 après qu'il ait traversé la membrane en sa zone hydrophile, mais leur rôle est aussi, sur le reliquat de liquide non expulsé qui est réaspiré vers le flacon dans la phase d'entrée d'air en compensation du liquide expulsé, de faciliter que sous la poussée de l'air il soit dirigé vers la zone hydrophile 22, en dégageant la zone hydrophobe centrale 23 pour l'air qui arrive ensuite dessus.

La surface de l'embase 3 présente par ailleurs des corrugations qui tendent à diviser finement toute veine de circulation d'air prenant naissance au sortir de l'embouchure du canal capillaire de l'embout, ce qui tend à réduire la vitesse à laquelle il traverse ensuite la membrane, alors même que celle-ci est poussée à l'écart de la surface transversale de l'embase de l'embout.

Dans la forme de réalisation préférée d'un embout ainsi réalisé conformément à l'invention, ce notamment dans le cas d'un embout compte-gouttes, les corrugations de division de toute veine d'air en circulation sont présentes sous la forme de sillons 32 relativement étroits et peu profonds, donc de fine section de passage, qui sont chacun en forme annulaire et répartis en disposition concentrique les uns par rapport aux autres autour du canal capillaire central de l'embout. Ces sillons 32 sont creusés en surface de l'embase d'embout, dans les secteurs de l'embase préservés par les rainures 31 de guidage du flux de liquide, là où la surface de l'embase d'embout est plutôt réservée à servir de support d'appui de la membrane lorsque celle-ci est poussée par la pression interne du flacon comprimé pour expulser du liquide.

On comprend qu'en fonctionnement, la configuration particulière de la surface de l'embout venant en regard de la membrane, joue un rôle dans l'organisation de la circulation des fluides, et ce non seulement en favorisant une alternance entre flux liquide et flux gazeux dans le canal central de l'embout, mais aussi en guidant les fluides sur leur trajet de retour comme le montrent les flèches sur la figure 4. Les flèches f1 illustrent que le reliquat de liquide non expulsé, rappelé en premier, est détourné d'un trajet axial direct et orienté vers la partie hydrophile de la membrane 22. On évite ainsi qu'il soit projeté sur la partie centrale de la membrane, où il tendrait à mouiller la matière de la membrane, de qualité hydrophobe en cet endroit. Le flux d'air aspiré vers le flacon dispose de ce fait d'un libre accès à la matière hydrophobe de la membrane en sa partie centrale 23, comme illustré par les flèches f2.

Revenant maintenant sur la figure 1, complétée par la figure 2, on observe d'autres détails de réalisation de la tête de distribution de liquide hors d'un flacon de conditionnement stérile qui, pour être en eux-mêmes classiques des flacons fabriqués industriellement par la Demanderesse, n'en sont pas moins des moyens participant à la mise en œuvre de la présente invention par la qualité de la préservation de la stérilité dans le flacon.

En ce sens on notera la présence des godrons périphériques externes 15 de la nacelle 4, qui assurent une étanchéité contre les bactéries avec le col du flacon 10 au niveau de l'insert poreux 8. On notera aussi la configuration du capuchon 6 qui est telle que, quand il est vissé (en 12) sur le col du flacon, il vient obturer l'embouchure externe du canal 18. Il a entre autres pour rôle d'assurer une chute de pression en aval de la membrane qui évite que celle-ci soit mouillée par le liquide contenu dans le flacon tant que la bague d'inviolabilité 26 n'a pas été fracturée pour une première utilisation (première expulsion d'une goutte de liquide).

Dans le même ordre d'idée, on notera encore la forme de la nacelle 8 à son extrémité amont, à l'intérieur du flacon. Son utilité se fera en premier lieu sentir dans des réalisations destinées à la distribution de collyres à particularités physico-chimiques tensioactives ou visqueuses, et dans de tels cas les moyens illustrés seront avantageusement exploités en combinaison avec des formes de mise en œuvre plus spécifiques de l'invention, à savoir celles prévoyant une membrane de porosité relativement grossière conduisant à une moindre qualité de protection par filtration des micro-organismes alors qu'une protection par effet biocide est élevée. Ces moyens résident dans la configuration formant des arches 13 autour d'une pastille centrale 11 et se disposant dans le flacon 2 au-delà de son col 10. Ils ont été amplement décrits dans la demande de brevet WO 2011/095877. Ils contribuent à une organisation de la circulation des fluides favorable aux besoins de la présente invention dans le cas des mêmes liquides.

Les résultats d'essai qui ont été rapportés plus haut témoignent d'une amélioration de la sécurité microbiologique dans la durée et dans l'exposition à des contaminations importantes qui ne pouvait s'attendre d'un simple emploi d'une membrane chargée de cations biocides. Ils ne pourraient pas plus s'attendre d'une telle membrane qui serait en outre partiellement hydrophile et partiellement hydrophobe dans la mesure où une telle membrane ne pourrait à elle seule assurer l'alternance des flux de liquide et d'air qui circulent du flacon vers l'extérieur et vice versa.

Dans le cas de l'invention, cette circulation alternée est assurée du fait que la membrane partiellement hydrophile et partiellement hydrophobe faisant interface entre l'intérieur et l'extérieur du flacon est combinée avec un canal capillaire d'expulsion de liquide et de rentrée d'air situé en aval de la membrane. Elle est aussi assurée en compléments par les autres moyens qui concourent de façon connue à la maîtrise de l'alternance des flux sous les effets de pression, et par là à la régularité et la reproductibilité des masses et volumes véhiculés.

Enfin, si le fait d'appliquer la membrane chargée d'ions biocides dans un flacon classique de la Demanderesse est déjà inventif par le rôle que l'on fait jouer dans le transport de la charge active au reflux de liquide créé à chaque opération de distribution d'une dose de liquide, il n'en reste pas moins que l'on ne parviendrait pas encore à obtenir les résultats démontrés tant qu'il ne s'y ajouterait pas la présence d'un insert qui soit réalisé poreux pour faire office de bouchon de fermeture non étanche du flacon, et le cas échéant, régulateur de flux par son caractère poreux comme il est classique, mais qui en outre soit constitué en une matière polymère présentant dans sa masse des sites anioniques ayant l'effet attracteur des cations métalliques que savent avoir notamment des sites carboxyliques.

Des différences de comportement dans les transferts ioniques peuvent en fait s'expliquer en considérant que la membrane est une pièce finement poreuse de grande étendue relative en travers du conduit de circulation des fluides et de faible épaisseur, alors que l'insert est lui de porosité relativement grossière et qu'il est épais, donc de relativement grande longueur le long du circuit de circulation des fluides. En considérant aussi que contrairement au canal capillaire du côté aval, cet insert occupe le col du flacon sur une section transversale relativement étendue, comme la membrane.

En outre, alors que dans la membrane les cellules individuelles du matériau ne se remplissent guère que soit de liquide soit d'air, on observera que dans l'insert les deux fluides sont présents simultanément au sein des cellules. Dès lors, l'oxygène de l'air peut avoir un effet sur les transferts de charges ioniques au sein des cellules. L'activité biocide en destruction des bactéries aérobies s'y exerce donc autrement que dans les cellules de la membrane. De plus, air et liquide viennent en contact avec une grande surface de matériau actif, en correspondance avec la surface spécifique de l'insert. L'utilisation des charges cationiques en destruction des bactéries n'en est que plus efficace.

Et clairement, un effet similaire ne peut avoir lieu au niveau du circuit situé en aval de la membrane, puisque là le matériau de l'embout est dense et imperméable tant au liquide qu'à l'air. En conséquence, même si ce matériau est à base d'un polymère ionique porteur d'ions argent au départ, ces ions doivent migrer vers la surface pour être actifs sur les fluides. La surface de contact avec les fluides au niveau de l'embout est longue mais de faible périmètre autour de la section du canal capillaire. En outre, elle est alternativement en présence soit d'air soit d'eau, y compris lors du reflux du reliquat de solution aqueuse non expulsé.

La différenciation des phénomènes impliquant les transferts des charges ioniques entre la membrane d'interface bifonctionnelle et l'insert poreux bouchant le flacon est d'autant plus nette que l'organisation de la circulation des fluides à travers eux est mieux contrôlée pour préserver que la membrane reste sèche en sa zone hydrophobe et que le reflux de liquide passe par sa zone hydrophile. Tant que le flacon est en stockage, avant la première utilisation consommant du liquide, la membrane reste sèche quelle que soit la position du flacon, grâce à une surpression assurée du côté aval par la fermeture étanche du canal capillaire ; cette surpression règne aussi en amont, de sorte à maintenir la membrane isolée de tout contact avec l'insert poreux.

Quoi qu'il en soit, il est un fait qu'entre les deux corps poreux que sont la membrane et l'insert poreux, il se crée en fonctionnement un lit d'ions mobiles qui sont prélevés de l'insert par le flux de liquide extrait du flacon à chaque opération de distribution, à partir d'ions biocides qui y ont été amenés par un reflux de liquide non distribué lors d'opérations de distribution de liquide précédentes. En pratique, les ions biocides restent ainsi cantonnés à des déplacements de l'un à l'autre des corps poreux. En aval de la membrane, le liquide expulsé n'est pas altéré.

Sans prétendre connaître la réalité des phénomènes qui se produisent à l'échelle des molécules et des charges ioniques, on peut penser à un mécanisme faisant intervenir la disponibilité des sites actifs directement accessibles pour contact avec les fluides en surface de la matière polymère, compte tenu de la grande surface spécifique et du grand volume de vide au niveau de l'insert et compte tenu de la faible épaisseur de la membrane. La membrane constitue une source primaire qui aura été chargée d'agent biocide à la fabrication suffisamment pour être capable de fournir largement la quantité d'ions utile pour satisfaire aux besoins de chaque application tout au long de la durée de vie du flacon jusqu'à épuisement de son contenu initial de liquide. L'insert, quant à lui, est déterminant à la fabrication comme source de sites actifs de charge complémentaire à celle des ions biocides. A partir du moment où le flacon a été ouvert pour une première opération de distribution, l'insert bouchant le flacon entre en fonction à la fois pour protéger l'espace stérile intérieur et pour constituer une source d'agent biocide secondaire, qui garde en réserve les ions qui y ont été amenés en fin d'une opération de distribution (phase d'aspiration d'air) jusqu'à ce qu'ils soient repris à une opération de distribution ultérieure. Ceux qui seront ainsi repris par le flux de liquide prélevé du flacon seront conduits jusqu'à la membrane et ils y seront retenus, exception faite toutefois de ceux qui auront été consommés au passage par des bactéries présentes dans l'air.

La haute qualité de la préservation de la stérilité constatée lors des essais de contamination forcée va bien au-delà des besoins spécifiques au cas des flacons de collyres et autre liquides ophtalmiques, que l'on a pris l'habitude de conserver sous protection de membranes filtrant les micro-organismes extérieurs. Elle montre par contre que la technique de l'invention conserve son intérêt en alternative aux méthodes classiques même dans ce cas, alors que d'une manière générale elle sera utile dans de nombreux cas d'application ne demandant pas ou n'autorisant pas une finesse de filtration bactérienne au niveau de la membrane. Egalement, on conçoit aisément que la mise en œuvre de l'invention peut se concrétiser sous des réalisations convenant à de grandes capacités de liquide et de grandes durées de vie en utilisation discontinue et/ou des doses et formes de diffusion très diverses pour le liquide expulsé du canal à circulation alternée des fluides, par de simples adaptations dimensionnelles des éléments constitutifs essentiels du dispositif suivant l'invention.

## Revendications

1. Dispositif de distribution d'un liquide aqueux stérile, par doses étagées dans le temps, depuis un espace amont fermé récepteur du liquide vers un espace aval ouvert par l'intermédiaire d'un canal capillaire débouchant à l'air ambiant, au travers d'une membrane d'interface ledit dispositif comprend une membrane d'interface réalisée pour partie de nature hydrophile et pour partie de nature hydrophobe, de telle sorte qu'en fonctionnement, lors de chaque opération de distribution d'une dose de liquide, les flux d'air et de liquide circulent alternativement dans le canal capillaire et qu'il se produit un reflux d'un reliquat de liquide non expulsé, **caractérisé en ce que** ladite membrane d'interface (7) est constituée en un matériau filtrant qui comprend en masse des cations métalliques biocides, **et en ce que** ledit dispositif comprend un insert poreux (8), perméable tant au liquide qu'à l'air, qui est disposé en amont de la membrane sur le trajet des fluides et qui est réalisé en un matériau comportant des sites de charges négatives aptes à attraire des cations métalliques biocides issus de ladite membrane.

2. Dispositif suivant la revendication 1, **caractérisé en ce que** lesdits cations métalliques biocides comprennent des cations de l'argent.

3. Dispositif suivant l'une des revendications précédentes, **caractérisé en ce que** lesdits cations métalliques biocides de la membrane sont supportés par des macromolécules minérales de type des zéolithes intégrées à la masse du matériau de base de la membrane.

4. Dispositif suivant l'une des revendications précédentes, **caractérisé en ce que** lesdits sites de charges négatives aptes à attraire des cations métalliques biocides sont des groupes anioniques carboxyles.

5. Dispositif suivant l'une des revendications 1 à 4, **caractérisé en ce que** ledit insert poreux a une masse volumique comprise entre 0,2 et 0,8 g.cm³.

6. Dispositif suivant l'une des revendications précédentes, **caractérisé en ce que** ledit insert est à base d'un polymère de polyoléfine, de préférence choisi parmi le polyéthylène, le polypropylène, et les copolymères d'éthylène ou de polypropylène avec jusqu'à 25 % d'homologues supérieurs d'acides carboxyliques ou d'esters.

7. Dispositif suivant l'une des revendications 1 à 6, **caractérisé en ce que** ledit insert est constitué d'une matière fibreuse compactée.

8. Dispositif suivant l'une des revendications précédentes, **caractérisé en ce que** lesdits sites de charges négatives aptes à attraire des cations métalliques biocides résultent d'une irradiation de l'insert poreux par des rayons de type bêta ou gamma en présence d'oxygène.

9. Dispositif suivant l'une des revendications précédentes, **caractérisé en ce que** le matériau constituant ladite membrane présente un diamètre de pores moyen compris entre 0,1 et 1 micromètre.

10. Dispositif suivant la revendication 9, **caractérisé en ce que** le matériau constituant ladite membrane présente un diamètre de pores moyen compris entre 0,4 et 0,8 micromètre.

11. Dispositif suivant l'une des revendications précédentes, **caractérisé en ce que** ledit canal capillaire est formé au sein d'un matériau intégrant des cations métalliques biocides, en particulier portés par des macromolécules minérales.

12. Flacon de conditionnement stérile d'un liquide aqueux à distribuer par doses étagées dans le temps, par expulsion d'une dose de liquide hors du flacon et rentrée d'air extérieur en compensation, **caractérisé en ce qu'**il est équipé d'un dispositif de distribution dudit liquide constitué suivant l'une des revendications 1 à 11, dont ledit insert (8) est monté en fermeture non étanche de l'intérieur du flacon, ledit espace fermé étant alors l'intérieur du flacon.

13. Flacon suivant la revendication 12, présentant une paroi à déformation élastiquement réversible pour assurer la rentrée d'air extérieur en compensation de toute dose de liquide expulsée du flacon ainsi que le retour à travers ledit dispositif de tout reliquat de liquide non expulsé, ladite membrane étant montée avec ledit insert poreux (8) dans ledit dispositif de distribution du liquide en association avec des moyens d'organisation de la circulation des fluides air et liquide à travers elle, et dans lequel ladite membrane est disposée à la base d'un embout compte-gouttes au sein duquel est ménagé le canal capillaire (18) d'expulsion des gouttes, face à une embase dudit embout dans laquelle sont ménagés des moyens de guidage respectifs de l'air aspiré depuis l'extérieur et de tout reliquat de liquide non distribué appelé à refluer vers la partie aval du conduit de circulation des fluides, qui tendent à diriger le flux d'air vers la partie hydrophobe de la membrane disposée de préférence au centre de ladite membrane et à répartir le liquide sur sa partie hydrophile.

14. Flacon suivant la revendication 12 ou 13, dans lequel ledit insert est déterminé pour participer à l'organisation de la circulation des fluides en constituant un régulateur de flux.

## Patentansprüche

1. Vorrichtung zur Abgabe einer sterilen wässerigen Flüssigkeit in zeitlich abgestuften Dosen ausgehend von einem stromaufwärtigen geschlossenen Raum zur Aufnahme der Flüssigkeit hin zu einem stromabwärtigen offenen Raum über einen Kapillarkanal, der in die umgebende Luft mündet, durch eine Grenzflächenmembran, wobei die Vorrichtung eine Grenzflächenmembran umfasst, die mit teilweise hydrophiler Beschaffenheit und mit teilweise hydrophober Beschaffenheit hergestellt ist, derart, dass bei der Betätigung bei jedem Vorgang zur Abgabe einer Dosis von Flüssigkeit die Luft- und Flüssigkeitsströmungen abwechselnd in dem Kapillarkanal zirkulieren und ein Rückströmen einer Restmenge an nicht ausgestoßener Flüssigkeit auftritt, **dadurch gekennzeichnet, dass** die Grenzflächenmembran (7) aus einem Filtermaterial besteht, das in der Masse biozide Metallkationen umfasst, und dadurch, dass die Vorrichtung einen porösen Einsatz (8) umfasst, der sowohl für die Flüssigkeit als auch für die Luft durchlässig ist, der stromaufwärts der Membran auf dem Weg der Fluide angeordnet ist und der aus einem Material besteht, das negative Ladungsstellen umfasst, die geeignet sind, biozide Metallkationen, die aus der Membran stammen, anzuziehen.

2. Vorrichtung nach Anspruch 1, **dadurch gekennzeichnet, dass** die bioziden Metallkationen Silberkationen umfassen.

3. Vorrichtung nach einem der vorhergehenden Ansprüche, **dadurch gekennzeichnet, dass** die bioziden Metallkationen der Membran von mineralischen Makromolekülen vom Typ der Zeolithe getragen werden, die in die Masse des Basismaterials der Membran integriert sind.

4. Vorrichtung nach einem der vorhergehenden Ansprüche, **dadurch gekennzeichnet, dass** die negativen Ladungsstellen, die geeignet sind, biozide Metallkationen anzuziehen, anionische Carboxylgruppen sind.

5. Vorrichtung nach einem der Ansprüche 1 bis 4, **dadurch gekennzeichnet, dass** der poröse Einsatz eine Volumenmasse von zwischen 0,2 und 0,8 g.cm³ aufweist.

6. Vorrichtung nach einem der vorhergehenden Ansprüche, **dadurch gekennzeichnet, dass** der Einsatz auf Basis eines Polyolefinpolymers ist, das vorzugsweise ausgewählt ist aus Polyethylen, Polypropylen und Ethylen- und Polypropylen-Copolymeren mit bis zu 25 % höheren Carbonsäure- oder Ester-Homologen.

7. Vorrichtung nach einem der Ansprüche 1 bis 6, **dadurch gekennzeichnet, dass** der Einsatz aus einem verdichteten Fasermaterial besteht.

8. Vorrichtung nach einem der vorhergehenden Ansprüche, **dadurch gekennzeichnet, dass** die negativen Ladungsstellen, die geeignet sind, biozide Metallkationen anzuziehen, aus einer Bestrahlung des porösen Einsatzes mit Strahlen vom Typ Beta oder Gamma bei Vorhandensein von Sauerstoff resultieren.

9. Vorrichtung nach einem der vorhergehenden Ansprüche, **dadurch gekennzeichnet, dass** das Material, das die Membran bildet, einen mittleren Porendurchmesser von zwischen 0,1 und 1 Mikrometer aufweist.

10. Vorrichtung nach Anspruch 9, **dadurch gekennzeichnet, dass** das Material, das die Membran bildet, einen mittleren Porendurchmesser von zwischen 0,4 und 0,8 Mikrometer aufweist.

11. Vorrichtung nach einem der vorhergehenden Ansprüche, **dadurch gekennzeichnet, dass** der Kapillarkanal in einem Material gebildet ist, das biozide Metallkationen integriert, die insbesondere von mineralischen Makromolekülen getragen werden.

12. Sterile Verpackungsflasche für eine wässerige Flüssigkeit zur Abgabe in zeitlich abgestuften Dosen durch Ausstoßen einer Dosis von Flüssigkeit aus der Flasche und Einzug von Außenluft als Ausgleich, **dadurch gekennzeichnet, dass** sie mit einer Vorrichtung zur Abgabe der Flüssigkeit nach einem der Ansprüche 1 bis 11 ausgestattet ist, deren Einsatz (8) als nicht dichter Verschluss des Inneren der Flasche montiert ist, wobei der geschlossene Raum dann das Innere der Flasche ist.

13. Flasche nach Anspruch 12, die eine reversibel elastisch verformbare Wand aufweist, um den Einzug von Außenluft als Ausgleich für jede aus der Flasche ausgestoßene Dosis von Flüssigkeit sowie die Rückkehr jeder Restmenge an nicht ausgestoßener Flüssigkeit durch die Vorrichtung zu gewährleisten, wobei die Membran mit dem porösen Einsatz (8) in der Vorrichtung zur Abgabe der Flüssigkeit in Verbindung mit Mitteln zur Organisation der Zirkulation der Luft- und Flüssigkeitsfluide durch sie hindurch montiert ist, und wobei die Membran an der Basis eines Tropfenzähler-Anschlussstücks, in dem der Kapillarkanal (18) zum Ausstoß der Tropfen eingerichtet ist, gegenüber einem Sockel des Anschlussstücks angeordnet ist, in dem jeweilige Mittel zur Lenkung der von außen angesaugten Luft und jeder nicht abgegebenen Restmenge an Flüssigkeit eingerichtet sind, deren Rückströmen hin zum stromabwärtigen Teil der Leitung für die Zirkulation der Fluide herbeigeführt wird, die dazu neigen, die Luftströmung hin zum hydrophoben Teil der Membran zu lenken, der vorzugsweise in der Mitte der Membran angeordnet ist, und die Flüssigkeit auf ihrem hydrophilen Teil zu verteilen.

14. Flasche nach Anspruch 12 oder 13, wobei der Einsatz dazu bestimmt ist, an der Organisation der Zirkulation der Fluide mitzuwirken, indem er einen Strömungsregler bildet.

## Claims

1. Device for dispensing a sterile aqueous liquid, by doses spaced out over time, from a closed upstream space accommodating a liquid to an open downstream space via a capillary channel opening to ambient air, through an interface membrane, said device comprises an interface membrane produced to be partially hydrophilic and partially hydrophobic, in such a way that in operation, during each operation for dispensing a dose of liquid, the flows of air and of liquid circulate alternately in the capillary channel and a back flow of non-expelled remaining liquid takes place, **characterized in that** said interface membrane (7) is constituted by a filtration material the mass of which comprises biocidal metal cations, **and in that** said device comprises a porous insert (8), permeable both to liquid and to air, which is arranged upstream of the membrane on the path of the fluids and which is made of a material containing negatively charged sites capable of attracting biocidal metal cations originating from said membrane.

2. Device according to claim 1, **characterized in that** said biocidal metal cations comprise silver cations.

3. Device according to one of the preceding claims, **characterized in that** said biocidal metal cations of the membrane are supported by mineral macromolecules of the zeolite type incorporated in the mass of the base material of the membrane.

4. Device according to one of the preceding claims, **characterized in that** said negatively charged sites capable of attracting biocidal metal cations are anionic carboxyl groups.

5. Device according to one of claims 1 to 4, **characterized in that** said porous insert has a volumetric mass density comprised between 0.2 and 0.8 g.cm³.

6. Device according to one of the preceding claims, **characterized in that** said insert is based on a polyolefin polymer, preferably chosen from polyethylene, polypropylene, and the copolymers of ethylene or of polypropylene with up to 25% higher homologues of carboxylic acids or esters.

7. Device according to one of claims 1 to 6, **characterized in that** said insert is constituted by a compacted fibrous material.

8. Device according to one of the preceding claims, **characterized in that** said negatively charged sites capable of attracting biocidal metal cations result from irradiation of the porous insert with rays of the beta or gamma type in the presence of oxygen.

9. Device according to one of the preceding claims, **characterized in that** the material constituting said membrane has a pore diameter comprised between 0.1 and 1 micrometre.

10. Device according to claim 9, **characterized in that** the material constituting said membrane has an average pore diameter comprised between 0.4 and 0.8 micrometre.

11. Device according to one of the preceding claims, **characterized in that** said capillary channel is formed within a material incorporating biocidal metal cations, in particular borne by mineral macromolecules.

12. Sterile packaging bottle for an aqueous liquid to be dispensed in doses spaced out over time, by expulsion of a dose of liquid out of the bottle and entry of outside air in compensation, **characterized in that** it is equipped with a device for dispensing said liquid constituted according to one of claims 1 to 11, said insert (8) of which is mounted as a non-sealing closure of the inside of the bottle, said closed space then being inside the bottle.

13. Bottle according to claim 12, having a wall that can be reversibly elastically deformed, in order to ensure the entry of outside air compensating for any dose of liquid expelled from the bottle as well as the back flow through said device of any non-expelled remaining liquid, said membrane being mounted with said porous insert (8) in said device for dispensing liquid in combination with means of organization of the circulation of the air and liquid fluids through it, and in which said membrane is arranged at the base of a dropper tip within which is arranged the capillary channel (18) for the expulsion of the drops, opposite a base of said tip in which are arranged respective means for guiding the air aspired from the outside and any remaining liquid that has not been dispensed and is required to flow back to the downstream portion of the duct for the circulation of the fluids, which tend to direct the airflow to the hydrophobic part of the membrane preferably arranged in the centre of said membrane and to distribute the liquid over its hydrophilic part.

14. Bottle according to claim 12 or 13, in which said insert is intended to participate in the organization of the circulation of the fluids by constituting a flow regulator.
